(19) Europäisches Patentamt
European Patent Office
Office européen des brevets

(11) Veröffentlichungsnummer: **0 050 870**
**B1**

# (12) EUROPÄISCHE PATENTSCHRIFT

(45) Veröffentlichungstag der Patentschrift: **01.08.84**

(51) Int. Cl.³: **C 07 C 87/02,** C 07 C 85/20, C 07 C 69/06, C 07 C 67/22

(21) Anmeldenummer: **81108899.6**

(22) Anmeldetag: **25.10.81**

(54) **Verfahren zur Herstellung von N-tert.-Alkylaminen oder Cycloalkylaminen und Estern der Ameisensäure.**

(30) Priorität: **27.10.80 DE 3040405**

(43) Veröffentlichungstag der Anmeldung: **05.05.82 Patentblatt 82/18**

(45) Bekanntmachung des Hinweises auf die Patenterteilung: **01.08.84 Patentblatt 84/31**

(84) Benannte Vertragsstaaten: **AT BE CH DE FR GB LI NL**

(56) Entgegenhaltungen:
**DE - B - 1 197 091**
**DE - B - 1 493 433**

(73) Patentinhaber: **Degussa Aktiengesellschaft, Weissfrauenstrasse 9, D-6000 Frankfurt am Main 1 (DE)**

(72) Erfinder: **Kleemann, Axel, Dr. Dipl.-Chem., Greifenhagenstrasse 9, D-6450 Hanau 9 (DE)**
Erfinder: **Lehmann, Bernd, Dr. Dipl.-Chem., Flurstrasse 5, D-6463 Freigericht 1 (DE)**
Erfinder: **Schalke, Peter, Dr. Dipl.-Chem., Südring 74, D-6458 Rodenbach (DE)**

**EP 0 050 870 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Aminen und Estern unter Umsetzung von Alkanolen mit Cyanwasserstoff in Gegenwart starker Säuren.

Es sind mehrere Verfahren zur Herstellung von N-tert.-Alkylaminen bekannt. In allen Fällen werden zunächst in einer ersten Verfahrensstufe N-tert.-Alkylformamide gewonnen. Aus diesen werden dann in einer zweiten Verfahrensstufe durch Hydrolyse die N-tert.-Alkylamine erzeugt, wobei gleichzeitig Ameisensäure entsteht.

Bekannt ist, dass bei der Umsetzung von Alkenen mit Cyanwasserstoff in Gegenwart von stark sauren Kondensationsmitteln, wie Schwefelsäure, Formamide entstehen und diese durch Hydrolyse mittels starker Säuren oder Basen in die entsprechenden Amine und Ameisensäure übergeführt werden. Beispielsweise wird aus Isobuten das N-tert.-Butylformamid und aus diesem neben Ameisensäure das N-tert.-Butylamin gebildet. Im Falle der Hydrolyse des Formamids mittels Chlorwasserstoffsäure wird aus dem Umsetzungsgemisch zunächst die Ameisensäure und dann nach Zugabe von Basen das Amin abdestilliert (DE-PS 870 856). Nachteilig ist bei diesem Verfahren, dass die Ausbeuten mässig sind und überdies die Ameisensäure in Form unreiner verdünnter wässriger Lösungen anfällt.

Es ist auch bekannt N-tert.-Alkylformamide durch Umsetzung tertiärer Alkohole oder verzweigter Alkene mit Cyanwasserstoff unter Verwendung von Ameisensäure als Kondensationsmittel zu erzeugen und die Formamide durch Hydrolyse mittels Alkali in die N-tert.-Alkylamine zu überführen (DE-AS 2 236 040). Bei diesem Verfahren sind die Umsetzungszeiten für die Bildung der Formamide sehr lang und die erzielten Raum-Zeit-Ausbeuten gering. Brauchbare Ausbeuten werden nur erreicht, wenn der Cyanwasserstoff und insbesondere die Ameisensäure in mehrfach molarem Überschuss eingesetzt werden. Diese im Überschuss angewendeten Substanzen können nur unter erheblichem Aufwand und überdies nur unvollständig zurückgewonnen werden.

Bekannt ist ausserdem, dass aus tert.-Alkyläthern die entsprechenden N-tert.-Alkylformamide entstehen, wenn die Äther mit Cyanwasserstoff in der 4fachen bis 50fachen Menge konzentrierter Säure umgesetzt werden und das Umsetzungsgemisch mit Wasser verdünnt wird (US-PS 2 518 156). Dieses Verfahren ist wegen der erforderlichen grossen Menge konzentrierter Säure sehr aufwendig.

Schliesslich ist auch bekannt, das durch Umsetzung von Alkenen mit Cyanwasserstoff in Gegenwart von Schwefelsäure erzeugte tert.-Butylformamid mittels Natriumhydroxyd zu hydrolysieren, zunächst aus dem Umsetzungsgemisch das tert.-Butylamin abzudestillieren, dann in dem restlichen Umsetzungsgemisch die Ameisensäure mittels Schwefelsäure freizusetzen und mit Methanol zu verestern und schliesslich den Ameisensäuremethylester abzudestillieren (US-PS 4 131 642). Bei diesem Verfahren ist die Ausbeute sowohl an dem tert.-Butylamin als auch an dem Ameisensäureester unbefriedigend.

Es ist nun ein Verfahren zur Herstellung von Aminen und Estern unter Umsetzung von Alkanolen mit Cyanwasserstoff in Gegenwart starker Säuren gefunden worden, das dadurch gekennzeichnet ist, dass tertiäre Alkanole oder Cyloalkanole in Gegenwart der bis zu etwa zweifachen molaren Mengen der starken Säuren mittels primärer oder sekundärer Alkanole zu N-tert.-Alkylaminen oder Cycloalkylaminen und Estern der Ameisensäure mit primären oder sekundären Alkanolen werden. Bei diesem Verfahren werden unmittelbar aus den Alkanolen die Amine und gleichzeitig die Ester der Ameisensäure gebildet. Sowohl die Amine als auch die Ester fallen mit ausgezeichneter Ausbeute an.

Das erfindungsgemässe Verfahren ist besonders geeignet für die Umsetzung von Alkanolen der allgemeinen Formel

$$\begin{array}{c} R_1 \\ | \\ R_2 - C - OH \\ | \\ R_3 \end{array}$$

in der $R_1$, $R_2$ und $R_3$ gleich oder verschieden sind und unverzweigte oder verzweigte, gegebenenfalls substituierte, Alkylgruppen mit 1 bis 10, vorzugsweise 1 bis 4, Kohlenstoffatomen, insbesondere Methylgruppen, bedeuten oder $R_1$ und $R_2$ gemeinsam mit dem C-Atom, an das sie gebunden sind, ein, gegebenenfalls substituierter, Cycloalkylring mit 5 bis 12, vorzugsweise 5 bis 8, Kohlenstoffatomen im Ring, insbesondere ein Cyclohexanring, sind, und $R_3$ Wasserstoff oder eine unverzweigte oder verzweigte, gegebenenfalls substituierte und gegebenenfalls mit $R_1$ oder $R_2$ zu einem Ring geschlossene, Alkylgruppe mit 1 bis 10, vorzugsweise 1 bis 4, Kohlenstoffatomen, insbesondere eine Methylgruppe, ist, mittels Alkanolen der allgemeinen Formel

$$R_4 - OH \qquad II$$

in der $R_4$ eine unverzweigte oder verzweigte Alkylgruppe mit 1 bis 10 Kohlenstoffatomen, vorzugsweise eine Propylgruppe, Isopropylgruppe oder Äthylgruppe, insbesondere eine Methylgruppe, ist, mit Cyanwasserstoff zu Aminen der allgemeinen Formel

$$\begin{array}{c} R_1 \\ | \\ R_2 - C - NH_2 \\ | \\ R_3 \end{array} \qquad III$$

in der $R_1$, $R_2$ und $R_3$ die zuvor angegebenen Bedeutungen haben, und Ameisensäureestern der allgemeinen Formel

$$\begin{array}{c} R_4 - O - CH \\ \quad\;\; \| \\ \quad\;\; O \end{array} \qquad IV$$

in der $R_4$ die zuvor angegebene Bedeutung hat.

Als tertiäre Alkanole kommen beispielsweise 2--Methylpentanol-(2), 2-Methylhexanol-(2), 3-Methylpentanol-(3), 3-Methylhexanol-(3), 2,3-Dimethylbutanol-(2), 2,3-Dimethylpentanol-(3), 2,5-Dimethylhexandiol-(2,5) und 2,4,4-Trimethylpentanol-(2) und als Cycloalkanole beispielsweise Cyclopentanol, Cyclohexanol, Cycloheptanol, Cyclooctanol, 4-tert.-Butylcyclohexanol, 2,3-Dimethylcyclohexanol-(1), 1-Methylcyclopentanol, 2-Methylcyclopentanol, 3-Methylcyclopentanol, 1-Methylcyclohexanol, 2-Methylcyclohexanol, 3-Methylcyclohexanol, 4-Methylcyclohexanol, 2,6-Dimethylcyclohexanol, Borneol, Terpineol, Menthol und 1-Adamantol in Frage. Mit besonderem Vorteil ist das erfindungsgemässe Verfahren für die Umsetzung des tert.-Butanols und des tert.-Amylalkohols geeignet.

Statt Cyanwasserstoff können Substanzen verwendet werden, die unter den Umsetzungsbedingungen Cyanwasserstoff liefern, beispielsweise Salze der Cyanwasserstoffsäure, wie Natriumcyanid.

Als starke Säuren werden beispielsweise Monoester der Schwefelsäure, zweckmässigerweise mit einem Alkanol, das der allgemeinen Formel

$$R_4 - OH \qquad II$$

entspricht, in der $R_4$ die zuvor genannten Bedeutungen hat, oder Sulfonsäuren, wie Methansulfonsäure oder Benzolsulfonsäure, eingesetzt. Besonders geeignet ist Schwefelsäure.

In welchen Mengenverhältnissen die Substanzen angewendet werden und welche Umsetzungsbedingungen, wie Temperatur und Druck, gewählt werden, richtet sich gegebenenfalls nach der Art der Substanzen.

Je Mol des tertiären Alkanols oder Cycloalkanols werden bis zu etwa 2 Mol der starken Säuren eingesetzt. Im allgemeinen ist es zweckmässig, etwa 0,8 bis 1,8 Mol der Säuren zu nehmen. Vorzugsweise werden 1,0 bis 1,3, insbesondere 1,05 bis 1,15, Mol der Säuren je Mol des Alkanols angewendet.

Das Mengenverhältnis tertiäres Alkanol oder Cycloalkanol zu Cyanwasserstoff kann weitgehend beliebig gewählt werden, jedoch ist es in den meisten Fällen vorteilhaft, nicht weniger als etwa 0,8 und nicht mehr als etwa 2,0 Mol Cyanwasserstoff je Mol des Alkanols zu nehmen. Vorzugsweise werden 1,0 bis 1,2 Mol Cyanwasserstoff je Mol des Alkanols angewendet. Anstelle von Cyanwasserstoff können diesem äquivalente Mengen der Cyanwasserstoff liefernden Substanzen eingesetzt werden. In diesem Fall wird zusätzlich eine entsprechende Menge starker Säure benötigt.

Zur Durchführung des erfindungsgemässen Verfahrens ist es erforderlich, ein primäres oder sekundäres Alkanol zuzusetzen. Dieses kann als solches oder mit den übrigen Substanzen, gegebenenfalls als Monoester an Schwefelsäure gebunden, eingebracht werden. Wenngleich die Menge dieses Alkanols weitgehend beliebig gewählt werden kann, ist es im allgemeinen zweckmässig, je Mol tertiäres Alkanol oder Cycloalkanol etwa 1 bis 4 Mol, vorzugsweise 1,1 bis 2,0 Mol, des primären oder sekundären Alkanols zu nehmen. Gegebenenfalls wird zu Anfang der Umsetzung nur eine Teilmenge, etwa 0,7 bis 1,2 Mol, und gegen Ende der Umsetzung die Restmenge des primären oder sekundären Alkanols eingebracht.

Es kann, insbesondere gegen Ende der Umsetzung, zweckmässig sein, Wasser zuzusetzen. Je Mol tertiäres Alkanol oder Cycloalkanol werden im allgemeinen bis etwa 1 Mol, in manchen Fällen bis etwa 4 Mol, Wasser angewendet.

Zweckmässig ist es im allgemeinen, bei Temperaturen etwa zwischen —30 und +100°C zu arbeiten. Vorteilhaft sind in den meisten Fällen Temperaturen zwischen 20 und 80°C. Es kann zweckmässig sein, die Umsetzung bei Temperaturen bis zu etwa 50°C zu beginnen und bei höheren Temperaturen zu beenden, wobei mit Vorteil zunächst Temperaturen gewählt werden, die unterhalb des Siedepunkts des Umsetzungsgemischs liegen, und im weiteren Verlauf das Umsetzungsgemisch zum Sieden gebracht wird.

Wenngleich der Druck weitgehend beliebig gewählt werden kann, ist es vorteilhaft, vom Nomaldruck nicht wesentlich abzuweichen. In manchen Fällen kann es wegen des Vorliegens flüchtiger Substanzen zweckmässig sein, bei einem der Temperaturen entsprechenden höheren Druck zu arbeiten.

Eine bevorzugte Verfahrensweise ist, die Ausgangssubstanzen, die Alkanole, den Cyanwasserstoff und die starke Säure, in wasserfreier oder weitgehend wasserfreier Form einzusetzen, die Umsetzung zunächst bei niedrigen Temperaturen, die unterhalb des Siedepunktes des Umsetzungsgemischs liegen, durchzuführen, dann gegebenenfalls Wasser und gegebenenfalls die restlichen Anteile des primären oder sekundären Alkanols zuzusetzen und schliesslich das Umsetzungsgemisch zum Sieden zu bringen.

Aus dem Umsetzungsgemisch kann das Amin und der Ester in an sich bekannter Weise abgetrennt und gewonnen werden, wobei sich die Verfahrensweise gegebenenfalls nach der Art der Substanzen richtet. Vorteilhaft ist es in vielen Fällen, beispielsweise bei der Herstellung des tert.-Butylamins oder tert.-Amylamins einerseits und des Ester der Ameisensäure mit Propanol-(1), Propanol-(2), Äthanol oder Methanol andererseits, zunächst den Ester aus dem Umsetzungsgemisch abzutreiben, dann im restlichen Umsetzungsgemisch mittels alkalisch wirkender Substanzen das Amin freizusetzen und schliesslich dieses abzutreiben.

*Beispiel 1*

Eine Lösung von 27,0 g (1,0 Mol) Cyanwasserstoff in 74,1 g (1,0 Mol) tert.-Butanol und 32,0 g (1,0 Mol) Methanol wurde im Verlauf von 30 Minuten in 111,2 g 97%ige Schwefelsäure (1,1 Mol) eingetragen. Die Mischung wurde währenddessen auf 20 bis 25°C, weiter 60 Minuten lang auf 50°C und schliesslich nach Zugabe von 5,7 g Wasser und 32,0 g Methanol 60 Minuten lang unter Rückfluss auf Siedetemperatur gehalten. Dann wurde der Ameisensäuremethylester abdestilliert. Die Ausbeute betrug 55 g, entsprechend 91%, bezogen auf den eingesetzten Cyanwasserstoff. Aus dem verbliebenen Umsetzungsgemisch wurde nach Zugabe von 100 g Wasser das Methanol und nach Zugabe von 196 g einer 45%igen wässrigen Natriumhydroxidlösung das tert.-Butylamin abdestilliert. Es wurden 62 g des Amins gewonnen, entsprechend 85%

Ausbeute, bezogen auf das eingesetzte tert.-Butanol.

*Beispiel 2*

Es wurde wie nach Beispiel 1 verfahren, jedoch wurden statt tert.-Butanol 88,1 g (1,0 Mol) tert.-Amylalkohol eingesetzt. Die Ausbeute an Ameisensäuremethylester betrug 54 g, entsprechend 90%, bezogen auf den eingesetzten Cyanwasserstoff, und die Ausbeute an tert.-Amylamin 74 g, entsprechend 85%, bezogen auf den eingesetzten Amylalkohol.

*Beispiel 3*

Es wurde wie nach Beispiel 1 verfahren, jedoch wurden 152 g 97%ige Schwefelsäure (1,5 Mol) und statt tert.-Butanol 114 g (1,0 Mol) 2-Methylcyclohexanol eingesetzt sowie 267 g der 45%igen Natriumhydroxidlösung zugefügt. Die Ausbeute an Ameisensäuremethylester betrug 43 g, entsprechend 89%, bezogen auf den eingesetzten Cyanwasserstoff. Nach Zugabe der Natriumhydroxidlösung bildeten sich zwei Phasen, von denen die obere 1-Methylcyclohexylamin war. Die untere Phase wurde mit Diäthyläther extrahiert, und der Extrakt wurde mit der oberen Phase vereinigt. Diese wurde dann mit Natriumsulfat getrocknet und zur Gewinnung des Amins destilliert. Der Siedepunkt des Amins war 142°C. Die Ausbeute an Amin betrug 81 g bei 91% Reinheit, entsprechend 65%, bezogen auf das eingesetzte 2-Methylcyclohexanol.

*Beispiel 4*

Es wurde wie nach Beispiel 1 verfahren, jedoch wurden 152 g 97%ige Schwefelsäure (1,5 Mol) und statt tert.-Butanol 100,2 g (1,0 Mol) Cyclohexanol eingesetzt sowie 267 g der 45%igen Natriumhydroxidlösung zugefügt. Nach Zugabe der Natriumhydroxidlösung wurde wie nach Beispiel 3 verfahren. Die Ausbeute an Ameisensäuremethylester betrug 52 g, entsprechend 86%, bezogen auf den eingesetzten Cyanwasserstoff, die Ausbeute an Cyclohexylamin 78 g bei 92% Reinheit, entsprechend 72%, bezogen auf das eingesetzte Cyclohexanol. Der Siedepunkt des Cyclohexylamins war 133°C.

*Beispiel 5*

Es wurde wie nach Beispiel 1 verfahren, jedoch wurden statt Methanol jeweils 46,1 g (1,0 Mol) Äthanol eingesetzt. Die Ausbeute an Ameisensäureäthylester betrug 64 g, entsprechend 86%, bezogen auf den eingesetzten Cyanwasserstoff, und die Ausbeute an tert.-Butylamin 62 g, entsprechend 85%, bezogen auf das eingesetzte tert.-Butanol.

*Beispiel 6*

Es wurde wie nach Beispiel 1 verfahren, jedoch wurden statt Methanol jeweils 60,1 g (1,0 Mol) Propanol-(2) eingesetzt. Die Ausbeute an Ameisensäureisopropylester betrug 89 g bei 90% Reinheit, entsprechend 91%, bezogen auf den eingesetzten Cyanwasserstoff, und die Ausbeute an tert.-Butylamin 64 g, entsprechend 87%, bezogen auf das eingesetzte tert.-Butanol.

*Beispiel 7*

Es wurde wie nach Beispiel 1 verfahren, jedoch wurde eine Lösung von 27,0 g Cyanwasserstoff in 74,1 g tert.-Butanol verwendet und in eine Mischung aus 69,3 g Dimethylsulfat und 55,6 g konzentrierter Schwefelsäure eingetragen und vor dem Erhitzen auf Siedetemperatur wurden 27,0 g Wasser und 32,0 g Methanol zugesetzt. Die Ausbeute an Ameisensäuremethylester betrug 56 g, entsprechend 92%, bezogen auf den eingesetzten Cyanwasserstoff, und die Ausbeute an tert.-Butylamin 67 g, entsprechend 91%, bezogen auf das eingesetzte tert.-Butanol.

**Patentansprüche**

1. Verfahren zur Herstellung von N-tert.-Alkylaminen oder Cycloalkylaminen und Estern der Ameisensäure unter Umsetzung von Alkanolen mit Cyanwasserstoff in Gegenwart starker Säuren, dadurch gekennzeichnet, dass tertiäre Alkanole oder Cycloalkanole in Gegenwart der bis zu etwa zweifachen molaren Menge der starken Säuren mittels primärer oder sekundärer Alkanole zu N-tert.-Alkylaminen oder Cycloalkylaminen und Estern der Ameisensäure mit primären oder sekundären Alkanolen umgesetzt werden.

2. Verfahren nach Anspruch 1, dadurch gekennzeichnet, dass 1,05 bis 1,15 Mol Säure je Mol tertiäres Alkanol oder Cycloalkanol angewendet wird.

3. Verfahren nach Anspruch 1 oder 2, dadurch gekennzeichnet, dass als Säure Schwefelsäure verwendet wird.

4. Verfahren nach Anspruch 1 bis 3, dadurch gekennzeichnet, dass aus dem Umsetzungsgemisch zuerst der Ameisensäureester und dann das Amin abgetrennt wird.

**Claims**

1. A process for the production of N-tertiary-alkylamines or cycloalkylamines and esters of formic acid by reacting alkanols with hydrogen cyanide in the presence of strong acids, characterised in that tertiary alkanols or cycloalkanols are reacted in the presence of up to about double the molar quantity of the strong acids and in the presence of primary or secondary alkanols to produce N-tertiary-alkylamines or cycloalkylamines and esters of formic acid with primary or secondary alkanols.

2. A process according to claim 1, characterised in that from 1.05 to 1.15 mols of acid are used per mol of tertiary alkanol or cycloalkanol.

3. A process according to claim 1 or 2, characterised in that sulphuric acid is used as acid.

4. A process according to claims 1 to 3, characterised in that first of all the formic acid ester is separated, and then the amine is separated from the reaction mixture.

**Revendications**

1. Procédé de préparation de N-tert.-alcoylami-

nes ou de cycloalcoylamines et d'esters de l'acide formique, par réaction d'alcanols avec l'acide cyanhydrique en présence d'acides forts, procédé caractérisé en ce que l'on transforme des alcanols tertiaires ou des cycloalcanols en présence de proportions pouvant être jusqu'à deux fois molaires de l'acide fort, au moyen d'alcanols primaires ou secondaires, en N-tert.-alcoylamines ou en cycloalcoylamines et en ester formique d'un alcanol primaire ou secondaire.

2. Procédé selon la revendication 1, caractérisé en ce que l'on utilise 1,05 à 1,15 mole d'acide par mole d'alcanol tertiaire ou de cycloalcanol.

3. Procédé selon l'une des revendications 1 ou 2, caractérisé en ce que comme acide on utilise l'acide sulfurique.

4. Procédé selon l'une quelconque des revendications 1 à 3, caractérisé en ce que l'on sépare du mélange réactionnel d'abord l'ester formique et ensuite l'amine.